Europäisches Patentamt

European Patent Office

Office européen des brevets

(19)

(11) EP 0 805 353 A2

(12) **EUROPEAN PATENT APPLICATION**

(43) Date of publication:
05.11.1997 Bulletin 1997/45

(51) Int Cl.6: **G01N 33/543**, G01N 33/546

(21) Application number: 97302998.6

(22) Date of filing: 01.05.1997

(84) Designated Contracting States:
DE FR GB IT

(30) Priority: 02.05.1996 JP 111380/96

(71) Applicant: Tosoh Corporation
Shinnanyo-shi, Yamaguchi-ken, 746 (JP)

(72) Inventors:
• Nagata, Yoshihiko
Sagamihara-shi, Kanagawa (JP)

• Arai, Takahiro
Sagamihara-shi, Kanagawa (JP)
• Iida, Hiroshi
Zama-shi, Kanagawa (JP)
• Isawa, Yuichi
Ayase-shi. Kanagawa (JP)

(74) Representative:
Kearney, Kevin David Nicholas et al
KILBURN & STRODE
30 John Street
London, WC1N 2DD (GB)

(54) **Immunoreaction agent and process for production thereof**

(57) An immunoreactive agent is provided which comprises one or more immunoreactive substances and/or immunoreactive components in a layer or layers formed on a carrier in the presence of a protection agent, and dehydrated and dried. A process for producing the immunoreactive agent is also provided. The agent in a dry state has excellent storage stability without deterioration of the response ability, and can be produced readily.

FIG. 1

**Description**

Background of the Invention:

Field of the Invention:

The present invention relates to an immunoreaction agent comprising an immunoactive substance in a layer or layers formed on a carrier, and a process for production thereof. More specifically, the present invention relates to an agent useful for quantitative and selective determination of micro-quantities of a substance in a human body fluid or the like, and a process for production of the agent.

Description of the Related Art:

Immunoassay is widely employed for detection or determination of micro-quantities of a substance (for example, thyroid markers such as insulin, and thyroid stimulating hormones; infectious disease markers such as IgE, and HBsAg; and tumor makers or markers) contained in biological samples such as serum and urine. In the immunoassay, the reaction of an antigen of bonding with a specific antibody (antigen-antibody reaction) occurs with high specificity even at an extremely low concentration. Therefore, the concentrations of antigen, antibody, or the like can be measured by utilizing this characteristic of the reaction. Many immunoassay methods have been developed. Among the immunoassay methods, labeling immunoassay is widely employed in which one of the antigen or antibody is labeled with a detectable substance.

Of the immunoassay methods, most widely employed is enzyme immunoassay (hereinafter referred to as "EIA"). A typical example of the EIA is a sandwich method. In the sandwich method, a first antibody is bonded to a carrier (e. g., magnetic beads) to immobilize the antibody (immobilized antibody). Separately, a second antibody is bonded with an enzyme for labeling the antibody with the enzyme (enzyme-labeled antibody). A sample such as serum or urine which contains an objective substance, namely an antigen to be measured, is added to a solution containing the immobilized antibody and the labeled antibody to cause the antigen-antibody reaction. Thereafter, the enzyme-labeled antibody bonded through the objective antigen to the carrier is separated from the non-bonded enzyme labeled antibody (B/F separation), and the quantity of the enzyme is measured.

The carrier used for the measurement is exemplified by paper disks of cellulose or the like (JP-A-61-21983), porous matters having capillaries (JP-A-59-90056, and JP-A-61-228354), and magnet-sensitive carriers. An example of the magnet-sensitive carrier is spherical thermoplastic resin particles carrying magnetic powder like ferrite and coated thereon with a polymer (JP-A-61-167866). With such a magnet-sensitive carrier, the measurement sensitivity can be increased by stirring the reaction liquid by oscillation of the magnetic field.

The immobilization of the antibody or antigen onto the carrier is conducted generally by chemical bonding using a coupling agent, or by physical adsorption. The antibody or the like immobilized on the carrier, and a labeled antibody bonded with an enzyme or the like are placed together in an appropriate reactor for formulation to provide an immunoreaction agent.

The components necessary for the immunoassay, for example, an antibody or an antigen immobilized on a carrier, or a labeled antibody or antigen are generally less stable in storage in a solution state than in dried form. Therefore, the components are generally dehydrated and dried for storage. It is said that of various dehydration methods, freeze-drying is the safest and the most superior method conventionally.

Examples of the methods include freeze-drying of a solution containing a monoclonal antibody and gelatin to produce a stable formulation with the antigen-bonding activity retained (JP-A-5-65233); and sealing of agents for EIA by a shaping carrier matrix formed from gelatin, and vacuum-drying (Japanese Kohyo 7-500417).

The freeze-drying process, however, is costly, and is not readily enlarged to a production scale because it is a batch process, and needs a large plant including the freeze-driers. Further, the series of the production steps takes a long time, including dispensation of the solution to be vacuum-dried into reaction cells, freezing, vacuum-drying, restoration to room temperature, and release of the pressure from the vacuum. For the above reasons, the freeze-drying process is not suitable for mass production.

In immunoassay with such an agent, a so-called all-in-one type agent is preferred which is a dehydrated and dried agent formulation containing all the necessary components for the reaction. This agent is dissolved in a buffer solution or the like, and a test liquid is added thereto to start the immunoreaction.

The all-in-one type agent can be produced by dispensing the necessary components in a solution state into reaction cells and freezing it. In the process, the necessary agent components are formulated in a state of contact or a mixture thereof. In the step of freeze-drying, some local concentration occurs unavoidably in the initial stage of the freezing, and the necessary components can interact with each other to impair the agent performance. In other words, the enzyme-labeled antibody or the like may be adsorbed non-specifically onto the carrier immobilizing an antigen or an antibody to increase measurement background and lower the sensitivity, or the labeling enzyme moiety and another component may interact to lower the enzyme activity or the response to impair the storage stability.

For decreasing the contact between the components to avoid the above disadvantages, one method is a multistage dispensation in which freezing is conducted after addition of each of the component solutions and the next solution is added thereon after the freezing of the preceding solution. However, this production process is complicated and time-consuming, and is not practical. Further, the above process involves another problem that the dispensation of the solutions containing the immobilizing carrier into cells for freeze-drying takes a long time generally, so that the production efficiency cannot readily be improved.

Separately from the above-described freeze-drying, another method of formulation is disclosed in which bovine serum albumin is added as a stabilizer to the immobilized agent, and the agent containing an antigen or antibody bonded to a carrier is dried by air-drying, aeration-drying, vacuum-drying, freeze-drying, or a like drying method (JP-A-5-66985). In this method, only the carrier having the antibody or antigen is dehydrated and dried for stabilization. Therefore, this method cannot provide the all-in-one type agent suitable for use in immunoassay apparatus.

In another analogous method, an immunoactive substance (antibody or antigen) immobilized on a carrier is immersed in a solution containing a sugar (lactose, sucrose, dextrin, etc.) or a protein (bovine serum albumin, water-soluble gelatin, etc.) as a stabilizer, and the mixture is air-dried (JP-A-5-41946). In a still another method, gelatin is added to an immunoassay agent solution containing an antigen, antibody, or the like bonded to magnetic particles (JP-A-3-115862) for storage stability (JP-A-5-180836). These methods stabilize only the immobilized agent components.

In the aforementioned storage stabilization by addition of gelatin, the agent which is in a solution state is required to be stored in a frozen state, and the frozen agent is required to be thawed or melted on use, disadvantageously.

In a still another method, an antibody is immobilized on a carrier like a microplate, and then treated with an aqueous solution containing albumin or gelatin as a protecting agent (JP-A-59-206716, and JP-A-61-241665). In still another method, a collagen degradation product containing a sensitive latex and a sugar solution are applied on a diagnosis test slide, and are air-dried at room temperature (JP-A-1-114757). These methods, similarly as the above methods, stabilize only a part of the immunoassay components by drying, and the other components still involve problems.

In such circumstances, the present invention is accomplished after comprehensive investigation on formulation of immunoassay agent employing a carrier, in particular magnetic particles, or the like.

Summary of the Invention:

The present invention provides an immunoreaction agent comprising one or more immunoactive substances and/or immunoreaction components formed on a carrier in a layer or layers in the presence of a protection agent mainly composed of gelatin or the like, and dehydrated and dried.

The present invention also provides a process for producing an immunoreaction agent, comprising forming a layer or layers of one or more immunoactive substances and/or immunoreaction components, on a carrier, in the presence of a protection agent mainly composed of gelatin or the like, and dehydrating and drying the layer or layers.

Brief Description of the Drawings:

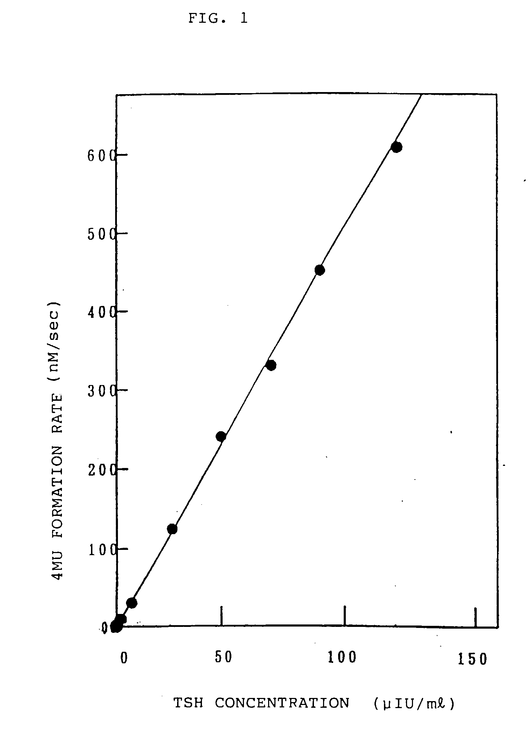

Fig. 1 shows dependency of 4MU formation rate on TSH concentration in enzyme immunoassay of TSH with the carrier prepared in Example 1 of the present invention.

Detailed Description of the Preferred Embodiment:

In the present invention, the necessary components for an immunoreaction are allowed to coat a carrier and are dehydrated and dried rapidly with minimum contact of the components with each other, in a large amount without employing a freeze-dryer to stabilize the agent for storage with retention of the agent performance.

According to the present invention, the final agent formulation can readily be prepared by immobilizing the necessary agent components on a carrier for storage stabilization and dispensing the carrier in a dry state into reaction cells. Thereby, the agent can be produced in a short time at low cost in a large quantity. The agent has excellent stability, and can be stored for a long term at room temperature or a lower temperature without freezing.

The agent of the present invention is produced, for example, through steps of immobilizing an antibody, an antigen, or a lectin onto a surface of a carrier like a particulate magnetic matter; treating it for blocking in a conventional manner; immersing it into a protection liquid mainly composed of gelatin or the like; removing, if necessary, any excess of the protection liquid; and dehydrating and drying the monolayer-coated carrier by centrifugal fluidization by supplying dry air or the like by means of a tumbling fluidized bed. The above coated carrier, in the present invention, may further be coated with an immunoactive component such as an enzyme-labeled antibody, or an inorganic salt, dissolved in the protecting liquid, and dehydrated and dried as above, successively by spray coating to form coating layers. In the multiple layer formation, a separation layer may be formed, if necessary, between the component layers to avoid the contact of the components with each other.

An immunoreaction component which is pulverizable, such as inorganic salts, can further be applied as a powder coat together with a suitable binder onto the above multilayer-coated immobilizing carrier. The binder may include gelatin, and hydroxypropylcellulose (HPC). In the present invention, a simple inorganic salt or the like participating in the immunoreaction may be allowed to coat the carrier in a monolayer. Otherwise, the carrier may be coated in multiple layers with such an immunoreaction-participating component and an immunoactive component in combination, or two or more immunoreaction-participating components.

The immunoreaction agent produced in such a manner is constituted, for example, of the immunoreaction components immobilized on a carrier such as a powdery magnetic matter, and coating layers of other components formed thereon. Since all the necessary immunoactive substances for the immunoreaction are immobilized by coating on the carrier in the present invention, an all-in-one type immunoreaction agent can be provided simply placing and sealing a required number of the coated carrier particles in reaction cells such as a plastic cups. This operation can be conducted by dispensing dry beads into cups by retaining the dry state by means of a dry dispensing machine to provide the final form of the agent rapidly and simply, which makes feasible the agent production in a short time in a large quantity.

The carrier useful in the present invention may be of any material which may be used in immunoassay, including inorganic particulate matter such as glass, and silica; and particulate synthetic high molecular compounds, and particulate cellulose derivatives. In particular, preferred are spherical carriers exemplified by particulate carriers mainly composed of thermoplastic resin such as polyethylene, polypropylene, ethylene-vinyl acetate copolymer, nylon, polystyrene, styrene-butadiene copolymers, and polyvinyl chloride or copolymers mainly composed thereof, and mixtures thereof. The particle diameter of the carrier is not limited, but is usually in the range of from 0.1 to 20 mm.

An example of a preferred carrier is the one which contains magnet-sensitive component, in the present invention. The magnet-sensitive component contained in the carrier includes soft ferrites such as manganese-zinc ferrite, and magnetites mainly composed of triiron tetraoxide. The particle diameter thereof is usually in the range of from 0.01 to 10 μm. The magnet-sensitive component may be incorporated in the carrier by mixing in the production process of the carrier composed of the above thermoplastic resin, or may be attached onto the surface of the carrier by adsorption, thermal fusion, or a like method.

The content (supporting amount) of the magnet-sensitive powdery matter in the carrier depends on the intensity of the magnetic field for carrier fluidization, or the degree of fluidity of the carrier, and is usually in the range of from about 1% to about 80%, preferably from 5% to 40% based on the weight of the beads.

Particles of balls of simple iron, stainless steel, or the like may be used as the carrier itself or the nucleus of the carrier particle in the present invention.

The carrier of the present invention can immobilize an immunoactive component chemically or physically as described above. The immobilization of the immunoactive component such as antibodies, antigens, and lectins is conducted specifically by physical adsorption, or chemical bonding by employing a coupling agent or the like (Seikagaku Jikkenhou (Experiments in Biochemistry) 11, (1989), pp.196-251, Tokyo Kagaku Dojin). In a particularly preferred method of preparation of the carrier, a polyparaxylylene film is formed on the surface of a carrier by chemical vapor deposition or a like method (JP-A-2-147861); the antigen, antibody, or lectin is immobilized on the film surface without additional chemical treatment by adsorption; and non-adsorption sites on the film surface are blocked with bovine serum albumin (hereinafter referred to as "BSA") or a like substance to prevent sufficiently nonspecific binding to the carrier.

The carrier thus prepared is immersed in a protection solution containing gelatin or the like as the main component for stabilization. Excess of the protecting gelatin solution is removed, if necessary, by a suitable method such as centrifuging, dehydration, or vacuum filtration. Then the carrier is dehydrated and dried by a centrifugal fluidization type granulator. The removal of the excess of the adhering protection solution is preferred for preventing aggregation of the carrier particles and promoting the later dehydration and drying by means of a granulation-dryer.

The gelatin for the protecting solution for the stabilization is preferably alkali-treated gelatin or acid-treated gelatin. Gelatin having a high water-solubility is particularly preferred, since the protection solution mainly composed of gelatin after drying is preferably dissolved again rapidly on dissolution of the dried agent without giving adverse effects to the immunoreaction.

The water-soluble gelatin is prepared by partial hydrolysis treatment of collagen, and, if necessary, subsequent purification. The gelatin is used preferably at a concentration ranging from 0.5 to 30 w/v%, more preferably from 5 to 15 w/v% in view of protection efficiency and production cost. The gelatin may be used in combination with another substance such as salts, buffering agents, amino acids, sugars, and proteins such as BSA. Such an additional substance is preferably soluble in a buffer solution having the pH around the neutral point, and may contain an antiseptic agent such as sodium azide in an appropriate amount. In the case where an enzyme-labeled antigen or antibody is contained as the immunoactive substance and $Mg^{2+}$ or $Zn^{2+}$ is necessary for activation of the enzyme, a salt thereof such as magnesium chloride or zinc chloride may be additionally used. The labeling enzyme is not specially limited, and may be any enzyme employed conventionally in immunoassay, including alkaline phosphatase (derived from bovine small intestine), peroxidase (derived from horseradish), and β-D-galactosidase (derived from Escherichia coli). The substance for the labeling may be luminescent and is not specially limited provided that it is used conventionally for luminescent immunoassay, including chemical luminescent substance such as acridinium derivatives.

As the immunoactive substance employed in the present invention, the antigen or antibody is selected suitably depending on the measurement object. Examples of the antigen include hormones separated and extracted from urine or serum, decomposition products thereof, and synthetic polypeptides corresponding to the components thereof. Example of the antibodies inclucle monoclonal antibodies collected from a cell culture or a mouse ascites by use of hybridoma, polyclonal antibodies prepared by purifying antiserum derived by sensitizing an animal with an antigen, and antibodies produced by recombinant techniques, and enzymatic decomposition products thereof such as F(ab')2, Fab', and Fab.

Use of an antigen or an antibody is decided suitably depending on the measurement system in which the immunoreaction agent in the present invention is to be used. As a agent for measurement of an antigen by a sandwich method, the immunoactive substance is exemplified by an immobilized antibody and labeled antibody against the antigen. As a agent for measurement of an antigen by a competition method, the immunoactive substance is exemplified by an immobilized antibody against the antigen, and the labeled one of the same antigen.

The granulation-drying machine which may be employed in the present invention may be a commercial rotation-fluidizing apparatus, and exemplified by New/Marumerizer (manufactured by Fuji Powdal K.K.), and Granulex (FREUND INDUSTRIAL Co., Ltd.).

For granulation, the carrier is placed in a granulation-drying machine, and the slit plate of the apparatus is rotated at such a rotation speed that the beads are kept in a centrifugally fluidized state. The drying and the granulation can be conducted efficiently by combination of a slit plate with a stirring blade rotated as necessary and with gas supply. The gas supplied is preferably dry air, and the relative humidity of the dry air is preferably not higher than 65%, more preferably not higher than 25%. The supplied gas may be an inert gas such as nitrogen, argon, or mixtures thereof in place of air. The gas feed rate is decided suitably depending on the amount of the beads, the relative humidity of the feed gas, the revolution speed of the stirring blade, the gas exhaustion rate, and other conditions, and is preferably in the range of from about 0.5 to about 5 $m^3$/min. The temperature of the feed gas is preferably in the range of from 4 to 60°C, more preferably not higher than 35°C.

The carrier prepared as above and coated with an immunoactive substance or an immunoreaction component is further coated with a still another immunoactive substance or immunoreaction component. This coating can be conducted by spray coating with a granulation machine as above, or powder-coating. For spray coating, the objective substance to be coated such as an enzyme-labeled antibody is added to a protection solution containing gelatin as the main constituent, and the solution is sprayed with air, an inert gas or a mixture thereof onto the carrier centrifugally fluidized in the granulating machine. The spraying conditions including the spraying rate are selected depending on the kind of the apparatus, the amount of the product, and other production conditions. For example, the spraying rate is preferably in the range of from 2 to 10 mL/min per kilogram of beads, and the amount of the sprayed liquid is in the range of from 30 to 300 mL per kilogram of beads.

The spray solution contains water-soluble gelatin or the like and an immunoactive substance, and may contain another substance as necessary. Otherwise, for example, a carrier having an antibody immobilized thereon is sprayed with an aqueous 1-30w/v% gelatin solution to add a protecting gelatin layer, further spray-coated with an aqueous solution containing 1-10w/v% BSA and 1-10w/v% gelatin, and still further spray-coated with an enzyme-labeled antibody to form a BSA-containing layer between the first antibody-immobilizing portion and the second enzyme-labeled antibody coating layer.

If necessary, for example, the carrier coated with multilayers of the immobilized antibody and the labeled second antibody may further be spray-coated with a solution containing various components to decrease interference of measurement by a serum component of the measured sample. Otherwise, the carrier may be powder-coated with an inorganic salt such as sodium chloride in an appropriate amount to decrease the influence on the immunoassay of a serum component in the measurement sample. Further, the surface of the coating layer containing the components necessary for the immunoassay formed on the carrier may further be coated with a component exhibiting no influence in the immunoassay. The formed outermost protection layer is effective in protecting the interior and preventing mutual contamination.

The powder coating can be conducted by pulverizing the inorganic salt in a size of several to several-ten microns, and feeding the pulverized inorganic salt in a powder state onto the surface of the carrier with simultaneous spraying of an aqueous 5-20w/v% water-soluble gelatin solution as a binder.

The particulate carrier prepared as above is placed in an appropriate particle number in a sealable reaction cell like a-plastic cup, and is sealed in a dry air atmosphere to provide an agent formulation which is storable for a long term. When the agent formulation is used in a sandwich method, the seal of the cup is broken; a measured amount of an objective sample (serum or urine of a patient, etc.) diluted with a suitable dilution solution is introduced into the cup;

the agent and the objective substance are allowed to react with agitation of the liquid in the cup at a constant temperature, usually 37±1°C, for a prescribed time; the carrier is washed for B/F separation; a solution of a detector substance eg a fluorescent substrate, for example, 4-methylumbelliferyl phosphate or the like is dispensed when alkaline phosphatase is employed as the labeling enzyme; measuring the change of the detected property eg. the fluorescence with time; and the measured data are compared with a calibration curve to measure the concentration of the objective substance in the sample for diagnosis.

The carrier of the present invention comprises multiple layers, the component in each of the layers being stabilized by dehydration and drying in the respective coating steps. Therefore, the carrier can be managed as a bulk, and can readily be produced discontinuously in a batch process. The carrier of the present invention can be dispensed speedily in a dry state into cells like plastic cups, much more speedily than a wet dispensation procedure, and is suitable for mass production. By this process, an agent formulation, such as a cup agent, can be produced by air-drying system without freeze-drying by simply sealing the cup in a dry air atmosphere.

Since the agent with the carrier is dried and stabilized for storage as a bulk agent, all-in-one type agent formulation can be produced simply by dry dispensation by means of a beads-dispensing and cup-sealing machine at any time and at any place.

The immunoreaction agent and the process for production thereof of the present invention have advantages as below:

(1) The antibody or antigen immobilized on the carrier can be dehydrated and dried rapidly and simply for storage stability with the bonding activity thereof retained without employing a lyophilizer;
(2) Not only the immunoactive substance immobilized onto the carrier but also other component (e.g., labeled antibody of labeled antigen) necessary as the immunoreaction agent or a component participating the immunoreaction can be dehydrated and dried rapidly and simply for storage stabilization with the bonding activity of the antigen or antibody retained in the same container as the dried matter of the above item (1) without employing a lyophilizer;
(3) The respective components necessary as the immunoreaction component can be dehydrated and dried independently without mutual contact, so that the drop of measurement sensitivity caused by increase of the background resulting from non-specific adsorption of the enzyme-labeled antibody to the carrier or other causes is prevented, and the decrease of the enzyme-activity or response caused by interaction between the antibody and other components is also prevented;
(4) A granulation-drying machine can be employed for the agent production in place of a freeze-drying apparatus, so that scale-up of the agent production can readily be conducted, and the agent can be produced simply in a small scale facility in a short time at a low cost in a uniform quality in a large amount;
(5) All the necessary components are dehydrated and dried, and sealed in a reaction cell in a stable state, so that freeze storage is not required, thawing is unnecessary on use, and the agent can be transported and distributed at room temperature;
(6) The agent can be supplied in an all-in-one type such that all the necessary components are sealed in a reaction cell to be set into an immunoassay apparatus;
(7) With the all-in-one type agent, the immunoreaction can be started by adding a dissolving liquid and a test sample liquid to the agent to dissolve rapidly the components coated on the carrier.

The present invention is described more specifically by reference to Examples without limiting the invention thereby.

Example 1

(Production of Carrier)

Into an ethylene-vinyl acetate copolymer (produced by Tosoh Corp.), was blended uniformly magnetite (produced by Titan Kogyo K.K.) which is composed of triiron tetraoxide having an average particle diameter of about 0.2 micron in an amount of 12 w/w%. The resulting blend was pelletized, and shaped into a spherical carrier of an average particle diameter of 1.5 mm containing a magnet-sensitive substance.

Onto 3 kg of this carrier, 60 g of dichloro-[2,2]-paracyclophane (produced by Daisan Kasei K.K.) was chemically vapor-deposited by means of a vacuum vapor deposition apparatus (manufactured by PARATECH COATING, INC.) to form a layer of a coating film of polymonochloroparaxylylene in a thickness of about 5 microns on the spherical carrier particles. The experiments below were conducted with this coated spherical carrier particles.

(Immobilization of Antibody on Carrier)

About 100000 particles (about 180g) of the carrier was suspended in 350mL of 20mM tris-hydrochloric acid buffer solution (pH=7.0). Thereto, 25 mg of anti-TSH (thyroid stimulating hormone) monoclonal antibody was added. The mixture was shaken at 25°C for 3 hours. The carrier was washed three times with 50mM tris-hydrochloric acid buffer solution (pH=8.0). The carrier was suspended in a 50mM tris-hydrochloric acid buffer solution (pH=8.0) containing lw/v% bovine serum albumin and treated at 53°C for 16 hours, for blocking.

(Production of Carrier of Present Invention by Granulation-Dryer)

About 100000 particles (about 180g) of the above blocked carrier was washed three times with 50mM tris-hydrochloric acid buffer solution (pH=8.0), and was immersed in 10w/v% gelatin solution (pH=7.0, water-soluble gelatin Type U produced by Nitta Gelatin K.K.), and kept standing still at 25°C for 30 minutes. Then the excess of gelatin was removed by centrifugation. The gelatin-treated carrier was dehydrated and dried by means of a granulation-dryer (NQ-LABO, manufactured by Fuji Powdal K.K.) for 5 minutes under the conditions of an air blow temperature of 32-35°C, an inlet air blow rate. of 0.5 m$^3$/min, a slit plate rotation rate of 200 rpm, an internal pressure of granulation-drying chamber of -30 mmH$_2$O caused by air exhaust.

The dried carrier was sprayed with 25w/v% gelatin solution for coating with gelatin. In the spraying, 15 mL of the gelatin solution was sprayed at a spray rate of 0.5 mL/min for about 30 minutes with the same granulation-dryer and under the same conditions as above except that the rotation rate of slit plate was changed to 300 rpm. After completion of the spraying, the carrier was dried further for about 2 minutes. The gelatin-coated carrier was sprayed with a BSA-containing solution for coating. In the spraying, 12 mL of 1w/v% gelatin solution containing 7w/v% BSA was sprayed at a spray rate of 0.5 mL/min for about 24 minutes with the granulation-dryer under the operation conditions of an air blow temperature of 24-27°C, an air blow rate of 0.5 m$^3$/min, a slit plate rotation rate of 300 rpm, and an internal pressure of the granulation-drying chamber of -30 mmH$_2$O caused by air exhaust. After completion of the spraying, the carrier was dried further for about 2 minutes.

About 90000 particles of (about 160g) the above carrier prepared above having a BSA-containing coating layer was sprayed with a gelatin solution which contains an anti-TSH monoclonal antibody-alkaline phosphatase conjugate capable of recognizing an epitope of TSH different from the epitope recognized by the aforementioned immobilized antibody. The spraying operation was conducted with 15 mL of 12.5w/v% gelatin solution (pH=7.0), which contained an alkaline phosphatase-labeled anti-TSH monoclonal antibody at a concentration of 100 mA (concentration corresponding to absorbance of 0.1 at wavelength of 280 nm) 75 mM magnesium chloride and 2.5mM zinc chloride, at a spray rate of 0.5 mL/min in about 30 minutes. The granulation-drying was conducted in the same manner as in the formation of the above BSA-containing layer.

(Enzyme immunoassay of TSH)

Twelve particles of the carrier prepared as above were placed respectively in a polypropylene cups of 10 mm in diameter and 20 mm in height containing therein 2 mg of sodium chloride, 150 nmol of magnesium chloride, and 5 nmol of zinc chloride. Thereto, a mixture of 100 μL of standard serum solution containing a known concentration of TSH and 50 μL of 0.01w/v% Triton X-100 (150 μL) was dispensed to respective cups. The carrier in the cup was stirred at 37°C for 40 minutes with a magnetic stirrer to allow the reaction to proceed.

After removal of the reaction liquid, the carrier particles were washed ten times with 50mM sodium chloride solution (pH=6.0) containing 0.04w/v% Tween 20 and 0.01w/v% Triton X-100. Thereto, was added 220 μL of a 1 mM solution (pH=10.0) of 4-methylumbelliferyl phosphate (4MUP) which is a substrate for alkaline phosphatase, and the reaction was allowed to proceed at 37°C.

The rate of formation of 4MU by hydrolysis of 4MUP by the enzyme was measured from 10 seconds after the addition of the substrate solution for 100 seconds by spectrometry at an excitation wavelength of 360 nm and a fluorescence wavelength of 450 nm. Fig. 1 shows the results.

Fig. 1 shows that the carrier of the present invention enables sensitive immunoassay. Incidentally, the minimum detection sensitivity of TSH is calculated to be 0.005 μIU/mL from the y-axis intercept and 2SD (standard deviation) thereof in Fig. 1.

Example 2

In a polypropylene cup of 10 mm in diameter and 20 mm in height, were placed 2 mg of sodium chloride, 150 nmol of magnesium chloride, and 5 nmol of zinc chloride. The propylene cup was dried, and therein, 12 particles of the carrier prepared as in Example 1 were put in. The cup was sealed in a dry air atmosphere. The sealed cup was subjected to an accelerated deterioration test at 40°C for 7 days in a thermostatic incubator. This test corresponds to cold storage for about one year or longer. As the control test, similar storage stability test was conducted at 4°C. The evaluation was conducted with TSH-containing standard human serum (TSH content: 50 μIU/mL) in the same manner as in Example 1. The remaining activity (antibody activity retention ratio, %) was derived as the percentage of the measured activity after storage at 40°C for 7 days relative to that at the start of the storage.

The results are shown in Table 1. The remaining activity is calculated according to the equation below:

$$\text{Remaining activity (\%)} =$$

$$[(\text{Rate after 7 days})/(\text{Rate at Start})] \times 100$$

("Rate" herein means the rate of 4MU formation.)

The rate at the start for the storage at 40°C was equal to that at the start at 4°C. Further, the same test was conducted with a standard serum solution not containing TSH. As the results, the rate did not increase from the start of the test even after the one-week accelerated storage test. Therefore, the background did not change and is stable, and increase of non-specific adsorption of the enzyme-labeled antibody to the carrier was not observed.

Table 1

| Treatment | Storage temperature | Remaining activity | | Increase of background | |
|---|---|---|---|---|---|
| | | Days of storage | | Days of storage | |
| | (°C) | 0 | 7 | 0 | 7 |
| Control Cold storage | 4 | 100 | 100 | none | none |
| Accelerated deterioration test | 40 | 100 | 98 | none | none |

Example 3

About 90000 particles of the carrier having the antibody immobilized thereon and blocked with BSA in the same manner as in Example 1 was washed three times with 50mM tris-hydrochloric acid buffer solution (pH=8.0). The carrier was immersed into 10w/v% gelatin solution (pH=7.0), and then dehydrated and dried by a granulation-dryer for 15 minutes under the drying conditions of an air blow temperature of 26-32°C, an air blow rate of 0.5 m$^3$/min, a slit plate rotation rate of 300 rpm, and an internal pressure of the granulation-drying chamber of -100 mmH$_2$O caused by air exhaust.

The dried carrier was sprayed with a BSA-containing solution for coating layer formation by using 15 mL of 5mM tris-hydrochloric acid buffer solution (pH=8.0) containing 7w/v% BSA and 1w/v% gelatin at a spraying rate of 0.5 mL/min under the conditions of an air blow temperature of 26-32°C, an air blow rate of 0.5 m$^3$/min, a slit plate rotation rate of 300 rpm, and an internal pressure of the granulation-drying chamber of -100 mmH$_2$O caused by air exhaust. After completion of the spraying, the carrier was dried further for about 2 minutes.

The BSA-coated carrier was spray-coated with a gelatin solution containing an alkaline phosphatase-labeled anti-TSH monoclonal antibody. The gelatin solution was a solution of the alkaline phosphatase-labeled anti-TSH monoclonal antibody at a concentration corresponding to absorbance of 0.1 at wavelength of 280 nm, 0.1M magnesium chloride, 8w/v% gelatin, and 2.5w/v% polyethylene glycol 4000 in 0.4M tris-hydrogen chloride buffer solution (pH-8.0). The spraying operation was conducted by use of 15 mL of the above gelatin solution at a spraying rate of 0.5 mL/min under the conditions of an air blow temperature of 26-32°C, an air blow rate of 0.5 m$^3$/min, a slit plate rotation rate of 300 rpm, and an internal pressure of the granulation-drying chamber of -100 mmH$_2$O caused by air exhaust. After completion of the spraying, the carrier was dried further for about 2 minutes.

On the carrier coated with the enzyme-labeled antibody, a separation layer was formed by spray-coating a gelatin solution. The spraying operation was conducted by use of 21 mL of 20w/v% gelatin solution in 0.1M tris-hydrogen chloride buffer (ph=8.0) at a spraying rate of 0.5 mL/min under the conditions of an air blow temperature of 26-32°C, an air blow rate of 0.5 m$^3$/min, a slit plate rotation rate of 300 rpm, and an internal pressure of the granulation-drying chamber of -100 mmH$_2$O caused by air exhaust. After completion of the spraying, the carrier was dried subsequently for about 3 minutes.

The carrier having the separation layer formed thereon was further coated with fine powdery sodium chloride (average particle diameter of about 45 microns). The powder-coating operation was conducted by spraying 20w/v% gelatin solution in 0.1M tris-hydrochloric acid buffer solution (pH=8.0) at a spray rate of 3.6 mL/min onto the carrier beads, and adding 13 g of fine powdery sodium chloride successively. The granulation-drying machine was driven in the same manner as in formation of the above separation layer formation.

Example 4

Twelve particles each of the carrier prepared in Example 3 were put in polypropylene cups of 10 mm in diameter and 20 mm in height. The cups were closed tightly by sealing in dry air atmosphere. The sealed cups were stored at 4°C or 40°C for 7 days, and evaluated in the same manner as in Example 2.

Table 2 shows the results. After storage at 40°C for 7 days, the background did not increase. Thus the storage stability was excellent.

Table 2

| Treatment | Storage temperature | Remaining activity | | Increase of background | |
|---|---|---|---|---|---|
| | | Days of storage | | Days of storage | |
| | (°C) | 0 | 7 | 0 | 7 |
| Control Cold storage | 4 | 100 | 100 | none | none |
| Accelerated deterioration test | 40 | 100 | 98 | none | none |

The invention also extends to a particulate immunoreaction agent in which each particle comprises a core, which may be magnetic, and which preferably has a polymeric surface or a polymeric surface layer, the surface of the core carrying a first layer comprising one or more immunoactive substances or one or more immunoreaction components, a first protective layer for stabilisation of the said substances or components. preferably of gelatin, formed over the said first layer, a second layer formed over the first protective layer, the said second layer containing one or more immunoreaction components if the first layer contained immunoactive substances and vice versa, and a second protective layer for stabilisation of the said substances or components, preferably of gelatin, formed over the said second layer, and optionally a further protective layer between the first and second layer, optionally containing bovine serum albumin, each of the layers being stabilised by drying before the next layer is formed.

**Claims**

1.  An immunoreaction agent, comprising one or more immunoactive substances and/or immunoreaction components in a layer or layers formed on a carrier in the presence of a protecting agent for stabilisation of said immunoreactive components, and dehydrated and dried.

2.  An immunoreaction agent as claimed in claim 1, wherein the immunoactive substance is an antigen, or an antibody, or an antibody labelled by a detectable substance.

3.  An immunoreaction agent as claimed in claim 2, wherein the detectable substance is an enzyme. a fluorescent substance, or a luminescent substance.

4.  An immunoreaction agent as claimed in claim 1, 2 or 3. wherein the protecting agent is water-soluble gelatin.

5.  An immunoreaction agent as claimed in any one of claims 1 to 4. wherein the carrier is particles of a polymer selected from polyethylene. polypropylene, ethylene-vinyl acetate copolymers, nylon, polystyrene, styrene-butadiene copolymers, polyvinyl chloride, and copolymers mainly composed thereof.

6.  An immunoreaction agent as claimed in any one of claims 1 to 5, wherein the carrier contains a magnetic substance.

7.  An immunoreaction agent as claimed in any one of claims 1 to 6, wherein the carrier having one or more immunoactive substances bonded thereon chemically or physically is further coated with other immunoactive substance and/or an immunoreaction component.

8.  A process for producing an immunoreaction agent, comprising forming a layer or layers on a carrier wherein one or more immunoreactive substances and/or immunoreactive components are contained in the layer or layers in the presence of a protecting agent for stabilisation of said immunoreactive substances and/or immunoreactive components, and dehydrating and drying the layer or layers.

9. A process for producing an immunoreaction agent according to claim 8, wherein the immunoactive substance is an antigen. or an antibody, or an antibody labelled by a detectable substance.

10. A process for producing an immunoreaction agent as claimed in claim 8 or claim 9, wherein the protecting agent is water-soluble gelatin.

11. A process for producing an immunoreaction agent as claimed in any one of claims 8 to 10, wherein the carrier is particles of a polymer selected from polyethylene, polypropylene, ethylene-vinyl acetate copolymers, nylon, polystyrene, styrene-butadiene copolymers, polyvinyl chloride, and copolymers mainly composed thereof.

12. A process for producing an immunoreaction agent as claimed in any one of claims 8 to 11, wherein the carrier contains a magnetic substance.

13. A process for producing an immunoreaction agent according to claim 9, wherein the carrier having one or more immunoactive substances bonded thereon chemically or physically is further coated with other immunoactive substances and/or an immunoreactive component, and the coated carrier is dehydrated and dried.

14. A process for producing an immunoreaction agent as claimed in any one of claims 8 to 13, wherein the layer or layers containing the immunoactive substance are formed on the carrier by means of a granulation-drying machine.

15. A process for producing an immunoreaction agent as claimed in any one of claims 8 to 14, wherein the carrier coated with the layer or layers containing the immunoactive substance is dehydrated and dried by means of a tumbling fluidizing machine.

16. A process for producing an immunoreaction agent according to claim 14 or 15, wherein the tumbling fluidizing machine is a rotation-fluidizing machine.

FIG. 1